# EUROPÄISCHE PATENTANMELDUNG

(11) **EP 3 477 300 A1**
(43) Veröffentlichungstag der Anmeldung: **01.05.2019**
(21) Anmeldenummer: 17001759.4
(22) Anmeldetag: 25.10.2017
(51) Int. Cl.: G01N 33/543, G01N 33/564

(54) **ZELLULOSE-BASIERTER IMMUNADSORBER**

(71) Anmelder: Euroimmun Medizinische Labordiagnostika AG, 23560 Lübeck (DE)
(72) Erfinder: LANGENHAN, Jana, 23923 Selmsdorf (DE); PROBST, Christian, 23909 Ratzeburg (DE)

(57) **Zusammenfassung**

Die Erfindung betrifft Immunadsorber zur spezifischen Entfernung von Autoantikörpern mittels Autoantigen aus Flüssigkeiten, wobei die Immunadsorber Zellulose oder Derivate davon als Trägermaterial aufweisen und Liganden über eine Affinitätsbindung an die Matrix gebunden sind; Verfahren zur Herstellung der erfindungsgemäßen Immunadsorber sowie Fusionsproteine zur Anwendung in einem Verfahren zur Behandlung von Autoimmunerkrankungen.

## Beschreibung

Die Erfindung betrifft neue Immunadsorber zur Entfernung von spezifischen Autoantikörpern mittels Autoantigenen zur Anwendung in der Therapie von Autoimmunerkrankungen, Verfahren zu ihrer Herstellung sowie Fusionsproteine zur Beladung der Immunadsorber.

Als Immunadsorption wird ein affinitätschromatographisches Verfahren bezeichnet, bei dem die spezifische Bindung zwischen Antikörper und Antigen zur Isolierung einer der Reaktionspartner aus Flüssigkeiten, wie z.B. Blut oder Blutplasma, genutzt wird.

Die Verwendung von Immunadsorbern zur extrakorporal, d.h. außerhalb des Körpers, stattfindenden Entfernung von pathogenen Substanzen oder Proteinen aus dem Blut wird im Stand der Technik u.a. zur Sepsistherapie durch die Entfernung von Komplementfaktoren und Lipopolysacchariden (EP 1 163 004), für die Behandlung von Entzündungen durch die Entfernung von Komplementfaktoren und Interleukinen (EP 1 802 342) und zur Entfernung von Endotoxinen (DE 199 38 394) beschrieben.

Des Weiteren bekannt ist die Immunadsorption, auch Immunapherese genannt, zur therapeutischen Entfernung von Antikörpern und Immunkomplexen bei Autoimmunerkrankungen oder Antikörper-vermittelter Transplantatabstoßung. Viele Autoimmunerkrankungen werden durch indikationsspezifische, pathogene Autoantikörper ausgelöst, die gegen körpereigene Proteine oder Strukturen gerichtet sind. Die Entfernung von Antikörpern aus dem Blut der Patienten ist ein Therapieansatz und daher finden Immunadsorber schon seit Jahren Verwendung in der Therapie. Erfahrungen mit der Anwendung der Immunapherese, d.h. der Entfernung von Antikörpern aus dem peripheren Blut, liegen bei folgenden Erkrankungen vor: Therapierefraktäre rheumatoide Arthritis, Hemmkörper-Hämophilie, idiopathische dilatative Kardiomyopathie, Nierentransplantation bei hochgradig sensibilisierten Transplantatempfängern und humoralen Abstoßungsreaktionen, adjuvante Therapie des Pemphigus und anderer bullöser Autoimmundermatosen, systemischer Lupus erythematodes, Myasthenia gravis/ Guillian-Barré-Syndrom, idiopathische thrombozytopenische Purpura, Erythrozytenaplasie durch AB0-Inkompatibilität nach allogener hämatopoetischer Stammzelltransplantation und Goodpasture-Syndrom (Zillikens et al., Empfehlungen für die Anwendung der Immunapherese bei der Therapie bullöser Autoimmundermatosen, JDDG, 10, Band 5, 2007).

Es gibt mehrere Immunadsorber auf dem Markt. Unterschieden werden dabei wiederverwendbare Adsorber (die aber nur für jeweils einen Patienten wieder genutzt werden) und Einmaladsorber. Weiterhin unterscheiden sich die bisher bekannten Systeme zur Adsorption von Immunglobulinen hinsichtlich des Liganden, der Affinität zu bestimmten Immunglobulinklassen, der Matrix und des Säulenvolumens.

Im Stand der Technik sind Einmaladsorber bekannt, die Dextransulfat, Staphylokokken-Protein A, Tryptophan oder Phenylalanin als Liganden verwenden. Adsorbiert werden mit diesen Adsorbern anti-dsDNA Antikörper, Lipide, Fibrinogen, Immunglobuline verschiedener Klassen (IgG, IgA und IgM) und Albumin (Zillikens et al., Empfehlungen für die Anwendung der Immunapherese bei der Therapie bullöser Autoimmundermatosen, JDDG, 10, Band 5, 2007).

Die Tryptophan-Immunadsorption zur Behandlung von Patienten mit einer Autoimmunerkrankung wird seit mehr als 20 Jahren eingesetzt. Anwendungsgebiete der Tryptophan-Immunadsorption sind u.a. Multiple Sklerose (MS), Guillian-Barré-Syndrom (GBS), Myasthenia gravis (MG), chronisch inflammatorische demyelinisierende Polyneuropathie (CIPD), Neuromyelitis optica (NMO), autoimmune Enzephalitiden, dilatative Kardiomyopathie, Antikörper-vermittelte Transplantatabstoßung, Goodpasture-Syndrom, fokal segmentale Glomerulosklerose (FSGS), systemischer Lupus erythematodes (SLE), Wegener-Granulomatose, Pemphigus-Erkrankungen, Hemmkörper-Hämophilie A und Thrombozytopenische Purpura (TTP/ITP) (Tryptophan-Immunadsorption, diAMED Medizintechnik). Der Tryptophan-Immunadsorber weist dabei eine Affinität zu Immunglobulinen der Klassen G, M, A und E auf, entfernt aber auch u.a. Fibrinogen und Albumin.

Weiterhin sind wiederverwendbare Immunadsorber bekannt, die als Liganden Staphylokokken-Protein A verwenden. Das Protein A des Bakteriums Staphylococcus ist eine Komponente der Zellwand des Bakteriums und hat die Eigenschaft, unselektiv Immunglobuline der Klasse IgG aufgrund seiner hohen Affinität zum Fc-Teil der IgG-Antikörper zu binden. Mit geringerer Affinität bindet es auch IgA- und IgM-Antikörper. Andere wiederverwendbare Immunadsorber nutzen als Liganden polyklonale anti-human-Antikörper vom Schaf oder auch synthetische Peptide wie PGMA146 und adsorbieren Antikörper der Klassen IgG, IgA und IgM (Zillikens et al., Empfehlungen für die Anwendung der Immunapherese bei der Therapie bullöser Autoimmundermatosen, JDDG, 10, Band 5, 2007).

Nachteilig an den bekannten technischen Lösungen ist, dass die bisher verwendeten Immunadsorber zu unspezifisch sind und neben den indikationsspezifischen Autoantikörpern wertvolle andere Blutbestandteile entfernt werden. Diese müssen dem Patienten aufwendig wieder zugeführt werden, was aber mit erheblichen Kosten und Risiken in der Langzeit-Anwendung verbunden ist.

Somit ist es bei diesen im Stand der Technik beschriebenen Immunadsorbern von Nachteil, dass Immunglobuline unabhängig von der Spezifität entfernt werden. Daher sind Adsorber entwickelt worden, die spezifische Autoantikörper adsorbieren. Bekannt ist ein Einmaladsorber, der als Ligand eine Kombination synthetischer Peptide (PDCM349+PDCM075) verwendet und damit zur Behandlung der dilativen Kardiomyopathie Autoantikörper gegen den beta1-adrenergen Rezeptor entfernt. Im Stand der Technik sind weiterhin spezifische Adsorber beschrieben, die für die Entfernung von Autoantikörpern gegen C1q bei der Behandlung des systemischen Lupus erythematodes oder für die Entfernung von Antikörpern gegen AB0-Blutgruppenantigene verwendet werden (Schmidt und Zillikens, Arch Dermatol Res (2010) 302: 241-253).

In diesem Zusammenhang beschreibt die WO2007/085240 das Problem der unspezifischen Entfernung von Immunglobulinen aus dem Blut von Patienten und schlägt vor, Autoantigene zur Immunadsorption von Autoantikörpern zur Behandlung von Autoimmunerkrankungen zu nutzen.

Zur Behandlung des bullösen Pemphigoids wird im Stand der Technik ein Immunadsorber für die spezifische Apherese der Autoantikörper mittels Autoantigen vorgeschlagen. Als Ligand eingesetzt werden drei verschiedene Formen von bakteriell hergestelltem BP180 (Typ XVII Kollagen), gegen das die Autoantikörper beim bullösen Pemphigoid gerichtet sind. Die Ergebnisse zeigen, dass die spezifische Immunadsorption zur Behandlung dieser Autoimmunerkrankung mittels Autoantigen vielversprechend ist (Mersmann et al., Arch Dermatol Res 2015: "Immunoadsorber for specific apheresis of autoantibodies in the treatment of bullous pemphigoid").

Die im Stand der Technik beschriebenen Immunadsorber unterscheiden sich weiterhin hinsichtlich des für die Matrix verwendeten Trägermaterials. Beschrieben ist die Nutzung von Sepharose, Silica-Gel, Polyvinylalkohol und Zellulose (Zillikens et al., Empfehlungen für die Anwendung der Immunapherese bei der Therapie bullöser Autoimmundermatosen, JDDG, 10, Band 5, 2007). Bei allen im Stand der Technik beschriebenen Immunadsorbern kann die biologisch aktive Substanz jedoch nur über eine chemische Kopplung auf die Matrix aufgebracht werden. Oftmals genutzt wird Bromcyan-aktivierte Sepharose (CNBr-Sepharose), an die sich Liganden mit primären Amino-Gruppen koppeln lassen. Mersmann et al. beschreiben die Verwendung von N-Hydroxysuccinimid (NHS)-aktivierte Agarose, NHSaktivierte Sepharose und verschiedene Ionenaustauscherharze (Mersmann et al., Arch Dermatol Res 2015: "Immunoadsorber for specific apheresis of autoantibodies in the treatment of bullous pemphigoid"). All diesen Trägermaterialien ist gemeinsam, dass sie verschiedene aktivierte chemische Gruppen aufweisen, die zu einer kovalenten Bindung der Aminogruppen führen.

Auch Zellulose wird im Stand der Technik mit Natriumperiodat, Epichlorhydrin und/oder N-Chlorcarbonyloxy-5-norbornen-2,3-dicarboximid aktiviert, um Liganden an diese Matrix koppeln zu können (Boeden et al., Journal of Chromatography, 552 (1991), 389-414).

Die chemische Aktivierung hat zur Folge, dass kovalente Bindungen mit den Liganden eingegangen werden. Kovalente Bindungen können jedoch auch gerade bei der Verwendung von Autoantigenen als Ligand im Bereich der antikörperbindenden Region erfolgen und damit die Antigen-Antikörper-Bindung beeinträchtigen. Grundsätzlich besteht immer die Gefahr, dass die Bindung des Autoantigens an die Matrix über eine Aminogruppe die Konformation des Autoantigens derart verändert, dass diese Antigene nicht mehr von den relevanten Autoantikörpern von Interesse erkannt werden.

Die aus dem Stand der Technik bekannten Verfahren zur Herstellung von Immunadsorbern sind sehr aufwendig und teuer. Hergestellte Liganden müssen gereinigt werden, die Trägermaterialien müssen vor der Kopplung mit den Liganden über chemische Verfahren aktiviert werden. Chemische Kopplungen sind meist mit der Verwendung toxischer Substanzen verbunden, mit entsprechenden Risiken für die Hersteller, die Patienten und Schwierigkeiten bei der Zulassung. Ein weiterer Nachteil ist, dass die Trägermaterialien im aktivierten Zustand nicht mehr sterilisierbar sind und eine Kopplung der Liganden unter aseptischen Bedingungen nicht mehr möglich ist. Die Möglichkeit zur aseptischen Kopplung ist aber sehr wichtig, insbesondere dann, wenn die Liganden zu instabil sind, um sie nach abgeschlossener Kopplung gemeinsam mit dem Trägermaterial zu sterilisieren.

Ausgehend von den bekannten Immunadsorbern und den zuvor geschilderten Problemen liegt der Erfindung die Aufgabe zugrunde, spezifische Immunadsorber zur Therapie von Autoimmunerkrankungen bereitzustellen. Es ist weiterhin eine Aufgabe der vorliegenden Erfindung, Immunadsorber bereitzustellen, in denen das Autoantigen zur Entfernung von Autoantikörpern derart gebunden vorliegt, dass die Aktivität in Bezug auf die Binding des Autoantikörpers erhalten bleibt. Es ist eine weitere Aufgabe der Erfindung, dass eine chemische Aktivierung der Trägermatrix vermieden wird. Weiterhin ist es eine Aufgabe der Erfindung, ein Verfahren zur Herstellung eines solchen Immunadsorbers bereitzustellen.

Diese Aufgaben werden durch den in den Patentansprüchen definierten Immunadsorber und das in den Patentansprüchen definierte Verfahren gelöst. Vorteilhafte Weiterbildungen der Erfindung sind Gegenstand der abhängigen Ansprüche und werden in der Beschreibung näher erläutert.

In einem ersten Aspekt richtet sich die vorliegende Erfindung auf einen Immunadsorber zur Entfernung von Autoantikörpern mittels Autoantigen aus Flüssigkeiten, dadurch gekennzeichnet, dass der Immunadsorber Zellulose oder Derivate davon als Matrix aufweist und ein Ligand über eine Affinitätsbindung an die Matrix gebunden wird.

Bevorzugt weist der Immunadsorber Zellulose-Beads als Matrix auf. In weiteren bevorzugten Ausführungsformen erfolgt die Affinitätsbindung über die Interaktion von Zellulose und Zellulose-bindenden Domänen des Liganden.

Bevorzugt liegt der Ligand als Fusionsprotein vor und weist eine Zellulose-bindende Domäne auf.

In weiteren bevorzugten Ausführungsformen weist das Fusionsprotein eine Zellulose-bindende Domäne und ein Autoantigen zur Adsorption von Autoantikörpern, die mit einer Autoimmunerkrankung assoziiert sind, auf.

In bevorzugten Ausführungsformen wird das Autoantigen über die Zellulose-bindende Domäne an die Zellulose immobilisiert und die Autoantikörper-bindende Domäne des Autoantigens so präsentiert, dass Autoantikörper hieran binden können.

In bevorzugten Ausführungsformen ist das Autoantigen ausgewählt aus der Gruppe bestehend aus Desmoglein 1 (Dsg1; UniProt: Q02413), Desmoglein 3 (Dsg3; UniProt: P32926), Bulöses-Pemphigoid-Antigen 2/Kollagen XVII (BP180; UniProt: Q9UMD9, speziell Domäne NC16A),

Aquaporin 4 (AQP4; UniProt: P55087), NMDA-Rezeptor Subtyp 1a (NR1a; UniProt: Q05586), Contactin-assoziiertes Protein 2 (CASPR2; UniProt: Q9UHC6), Leucin-reiches, in Gliomen inaktiviertes Protein 1 (LGI1; UniProt: 095970), Acetycholinrezeptor (AChR; UniProt: Q13702, P02708, P11230, Q07001, Q04844), Myelin-assoziiertes Glykoprotein (MAG; UniProt: P20916) und Varianten davon.

In bevorzugten Ausführungsformen ist die Zellulose-bindende Domäne ausgewählt aus der Gruppe bestehend aus Xyn10A aus Xylanase 10B aus *Cellulomonas fimi.*

In bevorzugten Ausführungsformen wird der erfindungsgemäße Immunadsorber für Apherese verwendet.

In bevorzugten Ausführungsformen wird der erfindungsgemäße Immunadsorber bei der Behandlung und Prophylaxe von Autoimmunerkrankungen ausgewählt aus der Gruppe bestehend aus Pemphigus foliaceus, Pemphigus vulgaris, Bullöses Pemphigoid, Schleimhautpemphigoid, Neuromyelitis optica, longitudinale extensive transverse Myelitis, rezidivierende Optikusneuritis, NMDA-Rezeptor-Enzephalitis, limbische Enzephalitis, Neuromyotonie, Morvan-Syndrom, Myasthenia gravis, paraproteinnämische anti-MAG Gammopathie (auch monoklonale IgM-Gammopathie mit anti-MAG-Reaktivität, paraproteinämische Neuropathie) und/oder dilative Kardiomyopathie und zur Verwendung bei der Behandlung einer Antikörper-vermittelten Transplantatabstoßung verwendet.

In einem zweiten Aspekt richtet sich die vorliegende Erfindung auf ein Verfahren zur Herstellung eines Immunadsorbers zur Adsorption von Autoantikörpern mittels Autoantigen, umfassend die folgenden Schritte: a) Bereitstellen eines Trägermaterials, b) Immobilisieren des Liganden auf diesem Trägermaterial, wobei der Ligand über eine Affinitätsbindung an das Trägermaterial gebunden wird.

In bevorzugten Ausführungsformen ist das Verfahren zur Herstellung dadurch gekennzeichnet, dass a) das Trägermaterial Zellulose aufweist und b) die Immobilisation des Liganden über die Interaktion von Zellulose und Zellulose-bindenden Domänen erfolgt.

In bevorzugten Ausführungsformen des Verfahrens zur Herstellung werden als Trägermaterial Zellulose-Beads verwendet.

In bevorzugten Ausführungsformen des Verfahren zur Herstellung liegt der Ligand als Fusionsprotein vor, umfassend eine Zellulose-bindende Domäne und ein Autoantigen zur Adsorption von Autoantikörpern.

In bevorzugten Ausführungsformen des Verfahren zur Herstellung werden das Trägermaterial und/oder der Immunadsorber vor und/oder nach der Beladung mit dem Liganden sterilisiert.

In einem dritten Aspekt richtet sich die vorliegende Erfindung auf in Fusionsprotein umfassend eine Zellulose-bindende Domäne und eine Autoantikörper-bindende Domäne zur Anwendung in einem Verfahren zur Behandlung von Autoimmunerkrankungen, dadurch gekennzeichnet, dass das Fusionsprotein über die Zellulose-bindende Domäne an Zellulose-Beads über Affinitätsbindungen gebunden vorliegt.

Die Erfindung betrifft in einem ersten Aspekt Immunadsorber zur Entfernung von Autoantikörpern mittels Autoantigenen aus Flüssigkeiten, wobei die Immunadsorber Zellulose oder Derivate davon als Trägermaterial aufweisen und die Liganden über eine Affinitätsbindung an die Matrix gebunden sind. Im Unterschied zu dem bekannten Stand der Technik werden die Liganden nicht kovalent an das Trägermaterial gebunden, die Bindung der Liganden an die Matrix kann ohne chemische Aktivierung des Trägermaterials erfolgen. Vorzugsweise werden als Trägermaterial Zellulose-Beads, Zellulose-Membranen oder Zellulose-Hohlfasern verwendet. Besonders bevorzugt werden Zellulose-Beads verwendet. Die Immobilisierung der Liganden an das Trägermaterial Zellulose erfolgt über eine Affinitätsbindung, vorzugsweise über die Interaktion von Zellulose und Zellulose-bindenden Domänen. In einer bevorzugten Ausführungsform der Erfindung liegt der Ligand als Fusionsprotein vor, welches eine Zellulose-bindende Domäne und eine Autoantikörper-bindende Domäne aufweist. In einer noch spezielleren Ausführungsform ist die Zellulose-bindende Domäne an ein Autoantigen zur Adsorption von Autoantikörpern, die mit einer Autoimmunerkrankung assoziiert sind, fusioniert. Über die Zellulose-bindende Domäne wird das Autoantigen an das Trägermaterial des Immunadsorbers derart immobilisiert, dass die Autoantikörper-bindende Domäne des Autoantigens so präsentiert wird, dass Autoantikörper hieran binden können. In einer bevorzugten Ausführungsform der Erfindung werden ein, zwei oder mehr verschiedene Autoantigene über Zellulose-bindende Domänen an das Trägermaterial des Immunadsorbers immobilisiert. In einer noch spezielleren Ausführungsform der Erfindung ist das Autoantigen ausgewählt aus der Gruppe umfassend: Desmoglein 1 (Dsg1; UniProt: Q02413), Desmoglein 3 (Dsg3; UniProt: P32926), Bullöses-Pemphigoid-Antigen 2/Kollagen XVII (BP180; UniProt: Q9UMD9, speziell Domäne NC16A), Aquaporin 4 (AQP4; UniProt: P55087), NMDA-Rezeptor Subtyp 1a (NR1a; UniProt: Q05586), Contactin-assoziiertes Protein 2 (CASPR2; UniProt: Q9UHC6), Leucin-reiches, in Gliomen inaktiviertes Protein 1 (LGI1; UniProt: 095970), Acetycholinrezeptor (AChR; UniProt: Q13702, P02708, P11230, Q07001, Q04844), Myelin-assoziiertes Glykoprotein (MAG; UniProt: P20916) und Varianten davon. Vorzugsweise ist das Autoantigen Dsg1 und/oder Dsg3 oder Varianten davon. Besonders bevorzugt ist die extrazelluläre Domäne von Dsg1 und/oder Dsg3. Die oben genannten Sequenzen der UniProt Datenbank (Stand: 1. Oktober 2017) sind hiermit durch Referenz in die vorliegende Beschreibung inkorporiert.

In einer bevorzugten Ausführungsform der Erfindung handelt es sich bei der Zellulose-bindenden Domäne um CeX/Xyn10A - Zellulose-bindende Domäne der Zellulose-Xylanase aus *Cellulomonas fimi,* Familie 2 (Genbank AEA30147.1) gemäß SEQ ID NO:1 und Varianten davon.

Eine bevorzugte Weiterbildung der Erfindung sieht die Verwendung des erfindungsgemäßen Immunadsorbers bei der Behandlung von Autoimmunerkrankungen vor, die einen erhöhten Titer bzw. Serumkonzentrationen ein oder mehrerer Autoantikörper aufweisen. In einer noch speziellere Ausführungsform der Erfindung wird der erfindungsgemäße Immunadsorber bei der Behandlung von Autoimmunerkrankungen verwendet, die ausgewählt sind aus der Gruppe umfassend: Pemphigus foliaceus, Pemphigus vulgaris, bullöses Pemphigoid, Schleimhautpemphigoid, Neuromyelitis optica, longitudinale extensive transverse Myelitis, rezidivierende Optikusneuritis, NMDA-Rezeptor-Enzephalitis, limbische Enzephalitis, Neuromyotonie, Morvan-Syndrom, Myasthenia gravis, paraproteinnämische anti-MAG Gammopathie (auch monoklonale IgM-Gammopathie mit anti-MAG-Reaktivität, paraproteinämische Neuropathie) und/oder dilatative Kardiomyopathie. Vorzugsweise wird der erfindungsgemäße Immunadsorber bei der Behandlung von Pemphigus vulgaris oder Pemphigus foliaceus verwendet.

Ein weiterer Aspekt der Erfindung betrifft ein Verfahren zur Herstellung eines Immunadsorbers gemäß den zuvor beschriebenen Ausführungsbeispielen zur Adsorption von Autoantikörpern mittels Autoantigen, wobei dieses Verfahren folgende Schritte umfasst.

Bereitstellen eines Trägermaterials,
a) Immobilisieren des Liganden auf diesem Trägermaterial,
wobei der Ligand über eine Affinitätsbindung an das Trägermaterial gebunden wird.

Eine bevorzugte Weiterbildung der Erfindung sieht vor, dass das Trägermaterial Zellulose, vorzugsweise Zellulose-Beads, aufweist und die Kopplung des Liganden an die Zellulose oder die Zellulose-Beads über Zellulose-bindende Domänen des Liganden erfolgt, d.h. ohne vorherige chemische Aktivierung des Trägermaterials.

In einer bevorzugten Ausführungsform der Erfindung sieht das erfindungsgemäße Verfahren Fusionsproteine als Liganden vor, umfassend eine Zellulose-bindende Domäne und ein Autoantigen. Die Immobilisierung der Autoantigene an das Trägermaterial des Immunadsorbers erfolgt über die an das Autoantigen fusionierte Zellulose-bindende Domäne.

In einer weiteren Ausführungsform der Erfindung werden das Trägermaterial und/oder der erfindungsgemäße Immunadsorber in dem erfindungsgemäßen Verfahren vor und/oder nach der Beladung mit dem Liganden sterilisiert. Bevorzugt erfolgt eine thermische Sterilisation durch Erhitzen im feuchten Zustand (Dampfsterilisation) und/oder die Sterilisation erfolgt physikalisch durch ionisierende Strahlen, wie z.B. UV-Strahlung, Röntgenstrahlung oder Gammastrahlung. Vorzugsweise erfolgt die Sterilisation durch eine thermische Behandlung in einem Autoklav. Eine bevorzugte Weiterbildung der Erfindung sieht vor, dass das Trägermaterial und/oder der erfindungsgemäße Immunadsorber nach der Beladung mit dem Liganden sterilisiert werden.

In einem weiteren Aspekt betrifft die Erfindung ein Fusionsprotein, welches eine Zellulose-bindende Domäne und eine Autoantikörper-bindende Domäne umfasst. Eine bevorzugte Weiterbildung der Erfindung sieht vor, dass dieses Fusionsprotein zur Anwendung in einem Verfahren für die Behandlung von Autoimmunerkrankungen gelangt, wobei das Fusionsprotein über die Zellulose-bindende Domäne an Zellulose-Beads über Affinitätsbindungen gebunden vorliegt. Eine bevorzugte Weiterbildung der Erfindung sieht vor, dass die Autoantikörper-bindende Domäne ein Autoantigen ist. In einer noch spezielleren Ausführungsform der Erfindung ist das Autoantigen ausgewählt aus der Gruppe umfassend: Desmoglein 1 (Dsg1; UniProt: Q02413), Desmoglein 3 (Dsg3; UniProt: P32926), Bullöses-Pemphigoid-Antigen 2/Kollagen XVII (BP180; UniProt: Q9UMD9, speziell Domäne NC16A), Aquaporin 4 (AQP4; UniProt: P55087), NMDA-Rezeptor Subtyp 1 a (NR1 a; UniProt: Q05586), Contactin-assoziiertes Protein 2 (CASPR2; UniProt: Q9UHC6), Leucin-reiches, in Gliomen inaktiviertes Protein 1 (LGI1; UniProt: 095970), Acetycholinrezeptor (AChR; UniProt: Q13702, P02708, P11230, Q07001, Q04844), Myelin-assoziiertes Glykoprotein (MAG; UniProt: P20916) und Varianten davon. Vorzugsweise ist das Autoantigen Dsg1 und/oder Dsg3 oder Variantendavon. Besonders bevorzugt ist die extrazelluläre Domäne von Dsg1 und/oder Dsg3. Die oben genannten Sequenzen der UniProt Datenbank (Stand: 1. Oktober 2017) sind hiermit durch Referenz in die vorliegende Beschreibung inkorporiert.

In einer bevorzugten Ausführungsform der Erfindung handelt es sich bei der Zellulose-bindenden Domäne um CeX/Xyn10A - Zellulose-bindende Domäne der Zellulose-Xylanase aus *Cellulomonas fimi,* Familie 2 (Genbank AEA30147.1) gemäß SEQ ID NO:1 und Varianten davon.

Der Begriff "Variante", wie hierin verwendet, umfasst Peptide und/oder Proteine, die eine Aminosäuresequenz, die zu der angegebenen Referenzaminosäuresequenz (Autoantigen oder Zellulose-bindenden Domäne, z.B. Dsg1, Dsg3, Zellulose-bindenden Domäne von CeX/Xyn10A gemäß SEQ ID NO:1) über deren Gesamtlänge oder einen Teil zu mindestens 70%, 71 %, 72%, 73%, 74%, 75%, 76%, 77%, 78%, 79%, 80%, 81 %, 82%, 83%, 84%, 85%, 86%, 87%, 88%, 89%, 90%, 90,5%, 91 %, 91 ,5%, 92%, 92,5%, 93%, 93,5%, 94%, 94,5%, 95%, 95,5%, 96%, 96,5%, 97%, 97,5%, 98%, 98,5% und 98,8% identisch oder homolog ist. Varianten weisen biologische Aktivität auf.

Eine Variante im Sinne der vorliegenden Erfindung umfasst auch Fragmente der angegebenen Referenzaminosäuresequenz (Autoantigen oder Zellulose-bindenden Domäne, z.B. Dsg1, Dsg3, Zellulose-bindenden Domäne von CeX/Xyn10A gemäß SEQ ID NO:1). "Fragment", wie hierin in Zusammenhang mit Autoantigenen oder Zellulose-bindenden Domänen verwendet, bezieht sich auf gegenüber dem Referenzpeptid N-terminal und/oder C-terminal um jeweils eine oder mehrere Aminosäuren verkürzte Polypeptide. In bevorzugten Ausführungsformen sind besitzen Fragmente mindestens 10 Aminosäuren, mindestens 20 Aminosäuren, mindestens 30 Aminosäuren, mindestens 50 Aminosäuren, mindestens 100 Aminosäuren oder mindestens 150 Aminosäuren und weisen die oben genannte Identität oder Homologie zum korrespondierenden Teil der Aminosäuresequenz des Referenzpeptids auf. Eingesetzt werden nur solche Fragmente, die biologische Aktivität aufweisen. "Biologische Aktivität", wie in diesem Zusammenhang verwendet, bedeutet insbesondere, dass die entsprechenden Peptide mindestens 30 %, mindestens 40%, mindestens 50 %, mindestens 60 %, mindestens 70 %, mindestens 80 %, mindestens 90 %, mindestens 95 % der spezifischen Bindeaktivität ihres Referenzpeptids aufweisen. Dies bedeutet im Fall der Autoantigene die spezifische Bindung an den Autoantikörper und im Fall der Zellulose-bindenden Domäne die spezifische Bindung an Zellulose oder deren Derivate.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, DJ. (1990) "Basic local alignment search tool." J. Mol. Biol. 215:403- 410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new genera- tion of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. Ferner möglich sind Sequenzvergleiche (Alignments) mit dem Computer- Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Erfindungsgemäß wird ein neuer Immunadsorber zur Entfernung von Autoantikörpern mittels Autoantigen aus Flüssigkeiten, wie z.B. Blut oder Blutplasma, auf Basis von Zellulose bereitgestellt, bei dem das Autoantigen mithilfe einer Zellulose-bindenden Domäne als Affinitätstag an die Zellulose bindet. Ein Vorteil dieser neuen Technik ist unter anderem, dass der Ligand ohne vorherige chemische Aktivierung des Trägermaterials an das Matrixmaterial Zellulose gebunden wird. Dadurch entfällt das Arbeiten mit teilweise gefährlichen Materialien und der Anfall von Sondermüll wird minimiert.

Die hierin verwendeten Begriffe, insbesondere die Begriffe "Autoantigen", "Antikörper", "Ligand", "Fusionsprotein", "Zellulose-bindende Domäne" und "Autoantikörper-bindende Domäne" werden sowohl im Singular als auch im Plural gleichbedeutend verwendet Das Wort "ein" und "eine" sind lediglich als unbestimmte Artikel zu verstehen und geben keine Anzahl oder Menge wieder.

Unter "Matrix" im Sinne dieser Erfindung wird das Trägermaterial des erfindungsgemäßen Immunadsorbers verstanden, an welches der Ligand immobilisiert wird. Vorzugsweise werden Zellulose-Beads, Zellulose-Membranen oder Zellulose-Hohlfasern als Trägermaterial verwendet. Besonders bevorzugt werden Zellulose-Beads. Der Begriff "Zellulose-Beads", wie hierin verwendet, bezieht sich auf Beads, die Zellulose umfassen oder aus dieser bestehen. In bevorzugten Ausführungsformen umfassen die Beads mindestens 20%, mindestens 30%, mindestens 40%, mindestens 50%, mindestens 60%, mindestens 70%, mindestens 80%, mindestens 90%, mindestens 95% oder mindestens 99% Zellulose. In alternativen bevorzugten Ausführungsformen ist Zellulose die Materialkomponente der Beads, die den größten molaren Anteil oder den höchsten Gewichtsanteil stellt.

"Zellulose Derivate", wie hierin verwendet, schließt Ester und Ether der Zellulose ein. In manchen Ausführungsformen werden die Zellulose Derivate der vorliegenden Erfindung durch Umsetzen von Allyl- oder Vinylmonomeren mit einem vernetzbaren Substituenten, wie vicinalen Dihydroxygruppen oder einer Vinylphosphonsäure, mit einem Cellulosederivat unter Verwendung eines Redoxsystems, umfassend Cer-Ionen und Salpetersäure generiert. Zellulose Derivate können im Wesentlichen durch den Abbau einer Zellulosekette zu Fragmenten mit einem gewichtsgemittelten Molekulargewicht von 2.000 oder weniger erhalten werden. Der Begriff Zellulose Derivat kann Substitutionen von einem oder mehreren Wasserstoffatomen in Zellulose-Hydroxylgruppen durch Alkylgruppen, vorzugsweise durch C₁-C₅, oder NO₂ umfassen.

Die im Immunabsorber verwendete Zellulose besitzt Poren. Verfahren zum Bestimmen der Porengrößen sind im Stand der Technik bekannt, z.B. über die Verwendung des Verteilungskoeffizienten (Kₐᵥ) von globulären (Standard)-Proteinen mit verschiedenen Molekulargewichten. Die Porengröße kann daher als Molekulargewicht angegeben werden, wobei das Molekulargewicht als Porengröße gilt, das unmittelbar unterhalb der Porenausschlussgröße liegt. In bevorzugten Ausführungsformen ist die Porengröße der Zellulose zwischen 3 - 1000 kDa, 10 - 900 kDa, 50 - 800 kDa, 100 - 500 kDa, 200 - 400 kDa. In anderen bevorzugten Ausführungsformen ist die Porengröße der Zellulose mindestens 50 kDa, mindestens 100 kDa, mindestens 110 kDa, mindestens 120 kDa, mindestens 130 kDa, mindestens 140 kDa, mindestens 150 kDa, mindestens 160, mindestens 170 kDa, mindestens 180 kDa, mindestens 190 kDa oder mindestens 200 kDa.

Der Begriff "immobilisiert", wie hierin verwendet, bedeutet, dass der Ligand über eine Affinitätsbindung an die Trägermatrix gebunden wird und somit fixiert vorliegt. Nicht umfasst sind kovalente Bindungen des Liganden an die Trägermatrix.

Der Begriff "Affinitätsbindung", wie hierin verwendet, bezieht sich auf nicht-kovalente Bindungen, wie z.B. Wasserstoffbrückenbindungen, elektrostatische Wechselwirkungen, Hydrophobe Wechselwirkungen und Van-der-Waals-Wechselwirkungen. Nichtkovalente Bindungen sind chemische Interaktionen zwischen Atomen, bei denen sich diese keine Elektronenpaare teilen.

In einer bevorzugten Ausführungsform wird der Immunadsorber zur Entfernung von Autoantikörpern mittels Autoantigen extrakorporal eingesetzt. "Extrakorporal" im Sinne dieser Erfindung bedeutet, dass der Immunadsorber außerhalb des menschlichen Körpers angeordnet ist. Das Blut oder Blutplasma fließt dabei durch den Immunadsorber an dem Trägermaterial mit den immobilisierten Autoantigenen entlang, welche eine hohe Bindungseigenschaft zu den Autoantikörpern aufweisen. Autoantikörper werden so durch die Autoantigene gebunden und das gereinigte Blut oder Plasma wird dem Patienten wieder zugeführt. Die Immunadsorption ist somit ein extrakorporales Therapieverfahren zur therapeutischen Entfernung von Autoantikörpern und Immunkomplexen bei Autoimmunerkrankungen oder Antikörper-vermittelter Transplantatabstoßung.

Unter Autoimmunerkrankungen sind im Kontext der vorliegenden Erfindung Erkrankungen zu verstehen, bei denen eine Immunreaktion gegen körpereigene Strukturen gerichtet ist. Es werden hierbei vom körpereigenen Immunsystem autoreaktive Immunzellen und/oder Antikörper gebildet, die gegen körpereigene Strukturen wie Zellen, Gewebe oder das Mikrobiom gerichtet sind, systemische oder organspezifische Schäden hervorrufen und somit dem Organismus in beliebiger Weise schaden können. Bei Autoimmunerkrankungen, die mit der Bildung von Autoantikörpern einhergehen, können die vom körpereigenen Immunsystem gegen körpereigene Strukturen gebildeten Antikörper entweder an der Entstehung der Autoimmunerkrankung ursächlich beteiligt sein oder aber den Verlauf ungünstig beeinflussen. Diese Antikörper sind "Autoantikörper" im Sinne dieser Erfindung. Somit kann der erfindungsgemäße Immunadsorber bei der Behandlung von Autoimmunerkrankungen eingesetzt werden, bei denen eine Absenkung der Autoantikörper-Spiegel eine Verbesserung der Symptomatik der Erkrankung bewirkt.

Unter "Autoantikörper-bindende Domäne" werden im Sinne dieser Erfindung Strukturen verstanden, die die Autoantikörper binden, wie z.B. Autoantigene oder gegen Autoantikörper gerichtete Antikörper. "Autoantigene" im Sinne dieser Erfindung sind Antigene, die von diesen Autoantikörpern gebunden werden. Ist das Antigen - die körpereigene Struktur, gegen die sich die Autoantikörper richten - bekannt, kann nahezu jedes Autoantigen als Ligand mit dem erfindungsgemäßen Immunadsorber eingesetzt werden.

Der erfindungsgemäße Immunadsorber kann verwendet werden bei der Behandlung von Autoimmunerkrankungen ausgewählt aus der Gruppe (nicht abschließend) umfassend Rheumatoide Arthritis/chronische Polyarthritis (Autoantigene: Kollagen II, zyklische citrullinierte Peptide, Immunglobulin G Fc-Fragment), Morbus Basedow (Autoantigen: Thyreotropin-Rezeptor), Myasthenia Gravis (Autoantigene: nikotinerger Acetylcholin-Rezeptor, muskelspezifische Tyrosinkinase), systemischer Lupus erythematodes (Autoantigene: nukleäre Proteine, Doppelstrang-DNA, SM-Ribonucleoprotein), medikamenteninduzierter Lupus erythematodes (Autoantigen: Histone), Goodpasture-Syndrom (Autoantigen: Typ IV-Kollagen), Sklerodermie (Autoantigen: DNA-Topoisomerase), CREST-Syndrom (Autoantigene: Zentromerproteine A, B und C), Polymyositis/Dermatomyositis (Autoantigen: Histidin-tRNA-Ligase), Granulomatose mit Polyangiitis (Autoantigen: Proteinase 3), Pemphigus vulgaris (Autoantigene: Desmoglein 1, Desmoglein 3), Sjögren-Syndrom (Autoantigene: Muscarinrezeptor M3, Ribonucleoproteine), Diabetes Mellitus Typ I (Autoantigene: Insulin, Inselzelloberflächen-Antigene, Glutamatdecarboxylase - überwiegend GAD65, aber auch GAD67, Thyrosinphosphatase), Zöliakie (Autoantigene: Retikulin, Endomysium, Gewebe-Transglutaminase), Kawasaki-Syndrom (Autoantigene: Oberflächenproteine von Endothelzellen mittelgroßer Gefäße), Pemphigus foliaceus (Autoantigen: Desmoglein 1), Bullöses Pemphigoid (Autoantigen: BP180), Schleimhautpemphigoid (Autoantigen: BP180), Neuromyelitis optica (Autoantigen: Aquaporin 4), longitudinale extensive transverse Myelitis (Autoantigen: Aquaporin 4), rezidivierende Optikusneuritis (Autoantigen: Aquaporin 4), NMDA-Rezeptor-Enzephalitis (Autoantigen: NMDA-Rezeptor Subtyp 1 a), limbische Enzephalitis (Autoantigene: CASPR2, LGI1), Neuromyotonie (Autoantigen: CASPR2), Morvan-Syndrom (Autoantigen: CASPR2), paraproteinämische anti-MAG Gammopathie (auch monoklonale IgM-Gammopathie mit anti-MAG-Reaktivität, paraproteinämische Neuropathie) (Autoantigen: Myelin-assoziiertes Glykoprotein = MAG, Glykoepitop HNK-1), Lambert-Eaton-Rook-Syndrom/pseudomyasthenisches Syndrom (Autoantigene: präsynaptische spannungsabhängige Calcium-Kanäle), autoimmune Gastritis (Autoantigene: intrinsischer Faktor, Natrium/Kalium-ATPase), Epidermolysis bullosa acquisita (Autoantigen: Kollagen VII), dilatative Kardiomyopathie, autoimmunhämolytische Anämie (Autoantigen: Oberflächenprotein von Erythrozyten), Stiff-Person-Syndrom (Autoantigen: Glutamatdecarboxylase), Neuromyotonie (Autoantigene: spannungsabhängige Kalium-Kanäle, nikotinerger AcetycholinRezeptor), Guillain-Barré-Syndrom (GBS)/Landry-Guillan-Barré-Strohl-Syndrom (Autoantigene: Galactocerebrosid, Gangliosid GM1), Miller-Fisher-Syndrom (Variante von GBS) (Autoantigen: Gangliosid GQ1b), Akute Motorische Axonale Neuropathie (Variante von GBS) (Autoantigen: Gangliosid GD3), Multifokale motorische Neuropathie (Variante von GBS) (Autoantigen: Gangliosid GM1), Sharp-Syndrom/Mischkollagenose (Autoantigene: nukleäre Proteine, Ribonukleoprotein-Komplexe), Dermatitis herpetiformis Duhring (Autoantigen: epidermale Transglutaminase), Antiphospholipid-Syndrom/Antiphospholipidantikörper-Syndrom/Cardiolipin-Antikörper-Syndrom (Autoantigene: Phospholipid-assoziierte Proteine), eosinophile Granulomatose mit Polyangiitis/Churg-Strauss-Syndrom (Autoantigen: Myeloperoxidase), mikroskopische Polyangiitis (Autoantigen: Myeloperoxidase), Antisynthetase-Syndrom (Autoantigen: Histidyl-tRNA-Synthetase), Bickerstaff-Enzephalitis (Autoantigen: Gangliosid GQ1 b), Lupus nephritis (Autoantigene: Phospholipide, C1 q) und/oder iodiopathische thrompozypoenische Purpura (Autoantigene: Oberflächenproteine von Thrombozyten, z.B. GpIIb/IIIa oder GpIb/XI). In bevorzugten Ausführungsformen kann der erfindungsgemäße Immunadsorber die oben genannten Autoantigene oder Varianten davon enthalten.

Ferner geeignet ist der erfindungsgemäße Immunadsorber auch für die Antikörper-vermittelte Transplantatabstoßung.

Vorzugsweise wird im erfindungsgemäßen Immunadsorber Zellulose als Trägermaterial verwendet. Besonders bevorzugt werden Zellulose-Beads als Matrix verwendet. Die Zellulose-Beads können dabei einen Partikeldurchmesser von 5- 1000 µm, 10 - 500 µm, 20 - 400 µm, 25 - 300 µm, 30 - 200 µm, 35 - 150 µm oder 40 - 130 µm aufweisen. Die Poren können eine Größe bilden, in die Substanzen mit einem Molekulargewicht von 3 - 500 kDa diffundieren können. Unter Porengröße ist im Sinne der Erfindung eine Ausschlussgröße zu verstehen, es handelt sich hier um einen Molekulargewichts-Ausschluss. Die Porengröße ergibt sich aus dem Grad der Quervernetzung der Zellulose. Besonders bevorzugt sind Zellulose-Beads mit einem Partikeldurchmesser von 80-100 µm, die eine Porengröße von 200 kDa bilden. Die Wahl der erforderlichen Porengröße ist im Wesentlichen abhängig von den gebundenen Liganden sowie von der mechanischen Stabilität. Die mechanische Stabilität wiederum hängt, wie auch schon die Porengröße, vom Grad der Quervernetzung ab.

Erfindungsgemäß wird der Ligand nicht kovalent an die Matrix gekoppelt, sondern über eine Affinitätsbindung, die über die Interaktion der Zellulose mit Zellulose-bindenden Domänen des Liganden erfolgt. Eine "Affinitätsbindung" im Sinne der Erfindung entsteht durch die Interaktion von Bindungspartnern bei Protein-Ligand-Wechselwirkungen aufgrund der Neigung der Moleküle mit dem anderen Molekül eine Bindung einzugehen. Je höher die Affinität, desto größer die Assoziationskonstante (auch Bindungskonstante). Diese Art der Bindung hat zufolge, dass die Matrix, das Trägermaterial des Immunadsorbers, chemisch nicht aktiviert werden muss. In bevorzugten Ausführungsformen ist die Dissoziationskonstante zwischen Antikörper und Autoantigen (bzw. Fragmenten davon) mindestens 10⁻⁴ M, mindestens 10⁻⁵ M, mindestens 10⁻⁶ M, mindestens 10⁻⁷ M, mindestens 10⁻⁸ M, mindestens 10⁻⁹ M oder mindestens 10⁻¹⁰ M.

Der erfindungsgemäße Ligand ist bevorzugt ein Fusionsprotein, umfassend eine Zellulose-bindende Domäne und ein Autoantigen. Die Zellulose-bindende Domäne des Liganden liegt bevorzugt als Affinitätstag vor, welcher an das Autoantigen fusioniert ist. Erfindungsgemäß wird dieser Affinitätstag auf genetischer Ebene anfusioniert. Dieser Tag kann sowohl C-terminal als auch N-terminal angebracht sein, jedoch darf die Bindungseigenschaft des Autoantigens nach Immobilisierung an das Trägermaterial über die Zellulose-bindende Domäne nicht beeinträchtigt sein. Um das zu vermeiden, können Linker-Sequenzen eingefügt werden. Dem Fachmann sind Methoden der Herstellung von Fusionsproteinen bekannt. Eine Übersicht über rekombinante Fusionstechnologien von Zellulose-bindenden Domänen sind beschrieben in C. Oliveira et al., Recombinant CBM-Fusion Technology - Applications Overview, Biotechnology Advances 33 (2015) 358 - 369.

Die Zellulose-bindenden Domänen, auch genannt Zellulose-bindende Module (CBM=cellulose-binding module), sind Protein-Domänen innerhalb Zellulose-modifizierender Enzyme oder Enzymkomplexe wie Glykosid-Hydrolasen, Gykosyl-Transferasen, Glykosyl-Esterasen, Expansinen oder Gerüstproteinen, die Bestandteil von Zellulosomen sind. Die Zellulose-bindenden Module werden aufgrund ihrer Aminosäuresequenz-Ähnlichkeit in mehrere Familien unterteilt. Zurzeit sind mehr als 80 Familien kohlenhydratbindender Module in der CAZy-Datenbank hinterlegt, die meisten von ihnen binden an Zellulose. Erfindungsgemäß können nur Zellulose-bindende Domänen eingesetzt werden, die eine Affinität zu kristalliner und/oder semikristalliner Zellulose aufweisen und irreversibel an die Zellulose binden. Die Zellulose-bindende Domäne sollte so ausgewählt sein, dass die Bindung an Zellulose nicht durch Serumbestandteile oder im Blut gelösten Substanzen, wie z.B. Glukose, gelöst werden könnte. Für eine effiziente Bindung an Zellulose kann die N-Glykolysierung der Zellulose-bindenden Domäne bei Expression in eukaryontischen Zellen verhindert werden, vorzugsweise durch eine Mutation von Asparagin zu Glutamin. Bevorzugt werden die Zellulose-bindenden Domänen ausgewählt aus der Gruppe umfassend

| Kürzel | Familie | Organismus | Protein |
|---|---|---|---|
| **CbhB, An01g11660** | CBM1 | *Aspergillus niger* | Zellobiohydrolase B |
| **Cbh-1** | CBM1 | *Athelia rolfsii* | Zellobiohydrolase |
| **Cel5A**, **BglC, BLi02088, BL01471** | CBM3 | *Bacillus licheniformis* | endo-β-1,4-Glucanase |
| **EGX** | CBM3 | *Bacillus pumilus* | endo-β-1,4-Glucanase |
| **CelD, Athe**_**0594** | CBM28 | *Caldicellulosiruptor bescii* | endo-β-1,4-Glucanase D |
| **Cel5A**, **CenD, Celf_1924** | CBM2 | *Cellulomonas fimi* | endo-β-1,4-Glucanase D |
| **Xyn10A, XynA**, **XylA, Xyl10A, CjXyn10A, CJA**_**2471** | CBM2 | *Cellvibrio japonicus* | Xylanase A/10A |
| **Cel5A**, **Cel5B, CelB** | CMB6 | *Cellvibrio mixtus* | endo-β-1,4-Glucanase B |
| **Cel7A, Cbh1, CtCbh1** | CBM1 | *Chaetomium thermophilum* | Zellobiohydrolase |
| **EngD** | CBM2 | *Clostridium cellulovorans* | endo-β-1,4-Glucanase Xylanase D |
| **CbpA** | CBM3 | *Clostridium cellulovorans* | Gerüstprotein |
| **CipA** | CBM3 | *Clostridium josui* | Gerüstprotein |
| **Cel5A**, **CelA** | CBM28 | *Clostridium josui* | endo-β-1,4-Glucanase |
| **Xyn11A, XynA** | CBM6 | *Clostridium stercorarium* | Xylanase A |
| **Xyn10B** | CBM1 | *Irpex lacteus* | endo-1,4-β-Xylanase |
| **Xyn10D** | CBM3 | *Paenibacillus curdlanolyticus* | Xylanase 10D |
| **Cel5C**, **PdCel5C** | CBM1 | *Penicillium decumbens* | Endoglucanase 5C |
| **Cel7D, CBH58, Cbhl.2, Cbhl-4, PcCel7D** | CMB1 | *Phanerochaete chrysosporium* | Zellobiohydrolase I-2 |
| **Cel6A, CbhII, Cbh2, PcCel6A** | CBM1 | *Phanerochaete chrysosporium* | Zellobiohydrolase II |
| **Xyn10A, Xynl, Xyn1, RmXyn10A, Rmar_1069** | CBM4 | *Rhodothermus marinus* | Xylanase |
| **Lic26A, Cel5E, CelH, EgH, CtCelH, CtCel5E, Cthe**_**1472** | CBM11 | *Ruminiclostridium thermocellum* | bifunktionale endo-β-1,4-Glucanase |
| | | | β-1,4-Xylanase H |
| **Cel9D/Cel9J+Cel44A**, **CelJ**, **Cthe**_**0624** | CBM30 | *Ruminiclostridium thermocellum* | Xyloglucanase J |
| | CBM44 | | prozessive Endoglucanase |
| **CipA, Cthe**_**3077** | CBM3 | *Ruminiclostridium thermocellum* | Gerüstprotein |
| **Clc16A, Lic16A, LicA, CtGlc16A, Cthe**_**2809** | CBM4 | *Ruminiclostridium thermocellum* | β-1,3-1,4-Glucanase |
| **Lic26A, Cel5E, CelH, EgH, CtCelH, CtCel5E, Cthe**_**1472** | CBM11 | *Ruminictostridium thermocellum* | bifunktionale endo-β-1,4-Glucanase |
| **1,3Gal43A, Ct1,3Gal43A, CtGH43, Cthe**_**0661** | CBM13 | *Ruminiclostridium thermocellum* | [Arabinogalactan] Exo-β-1,3-Galactanase |
| **Cthe**_**3095** | NC | *Ruminiclostridium thermocellum* | |
| **Man5A, CtMan5A, Cthe**_**0821** | CBM32 | *Ruminiclostridium thermocellum* | β-Mannanase |
| **Cthe**_**0246** | CBM35 | *Ruminiclostridium thermocellum* | Dockerin |
| **Cthe**_**2191** | CBM48 | *Ruminiclostridium thermocellum* | |
| **CipB** | CBM3 | *Ruminiclostridium thermocellum* | Zellulosom S1 Untereinheit |
| **Cbh9A, CbhA, Cthe**_**0413** | CBM3 | *Ruminiclostridium thermocellum* | Zellobiohydrolase |
| **Cel5H**, **Sde**_**3237** | CBM6 | *Saccharophagus degradans* | prozessive endo-β-1,4-Glucanase 5H |
| **SICel9C1, SIGH9C1, Cel8, TomCel8** | CBM49 | *Solanum lycopersicum* | endo-β-1,4-Glucanase 8 |
| **StCBM64C, Spith**_**0373** | CBM64 | *Spirochaeta thermophila* | |
| **AbfB2, AbfB-2, PfAbfB2** | CBM1 | *Talaromyces funiculosus* | α-L-Arabinofuranosidase |
| **XynX** | CBM9 | *Thermoanaerobacterium sp*. | Xylanase X |
| **Xyl11A, Xyn11A, XynA**, **TfxA, Tfu_1213** | CBM2 | *Thermobifida fusca* | Xylanase A |
| **Cel48A, E6**, **CelF, TfCel48A, Tfu**_**1959** | CBM2 | *Thermobifida fusca* | β-1,4-Exozellulase |
| **Eg-1** | CBM1 | *Trametes hirsute* | Endoclucanase |
| **Cel12A**, **Egl3**, **EG3, EGIII, ThEG3, ThEGIII** | | *Trichoderma harzianum* | endo-β-1,4-Glucanase 3/III |
| **Cel7A**, **Cbhl**, **TrCel7A**, **HjCel7A**, **TreCel7A** | CBM1 | *Trichoderma reesei* | Zellobiohydrolase I Chitosanase |
| **Cel5A**, **Eg2, EGII, Egl2**, **TrEGII, TrCel5A** | CBM1 | *Trichoderma reesei* | endo-β-1,4-Glucanase |
| **Cel6A, CBHII**, **Cbh2, TrCel6A, HjCel6A, TrCbh2** | CBM1 | *Trichoderma reesei* | Zellobiohydrolase II |
| **EG1, EGI** | CBM1 | *Volvariella volvacea* | Endoclucanase I |
| **EngXCA**, **XAC0612** | CBM2 | *Xanthomonas axonopodis pv. citri* | Endoglucanase |
| **Cex/Xyn10A** | CBM2 | *Cellulomonas fimi* | Xylanase B |

Besonders bevorzugt wird die Zellulose-bindenden Domäne der Zellulase-Xylanase/Xylanase B (Cex/Xyn10A, Familie 2) aus *Cellulomonas fimi* und Varianten davon.

Ein weiterer Aspekt der Erfindung sieht ein Fusionsprotein vor, ein rekombinantes Polypeptid umfassend eine oder mehrere Zellulose-bindende Domänen und eine oder mehrere Autoantikörper-bindende Domänen. Das erfindungsgemäße Fusionsprotein weist die Merkmale des zuvor beschriebenen Liganden auf und wird als solcher im Immunadsorber gemäß den zuvor beschriebenen Ausführungsbeispielen eingesetzt. Vorzugsweise ist die Autoantikörper-bindende Domäne ein Autoantigen, welches an einen Autoantikörper im Sinne der Erfindung bindet. Das Fusionsprotein liegt im Immunadsorber an das Trägermaterial gebunden vor. Es wird über die Zellulose-bindende Domäne an der Zellulose immobilisiert, und zwar derart, dass die Antikörper-bindende Domäne des Autoantigens frei ist und die entsprechenden Autoantikörper binden kann. Ferner kann das Fusionsprotein Linker-Sequenzen umfassen. Methoden zur Herstellung eines rekombinanten Proteins sind dem Fachmann bekannt und zum Beispiel beschrieben in J Sambrook et al., 1989, Molecular Cloning, CSH oder in Brown TA et al., 1986, Gene Cloning - an introduction, Chapman & Hall. Bevorzugt umfasst das erfindungsgemäße Fusionsprotein 1) die Zellulose-bindenden Domäne Cex/Xyn10A aus *Cellulomonas fimi* (Genbank: AEA30147.1) gemäß SEQ ID NO:1 oder Varianten davon und 2) ein Autoantigen. Bevorzugt ist das Autoantigen ein Protein wie oben beschrieben oder eine Variante davon. Besonders bevorzugt umfasst das Fusionsprotein das Autoantigen Desmoglein 1 und/oder Desmoglein 3 und/oder Variantendavon. Besonders bevorzugt umfasst das Fusionsprotein die extrazelluläre Domäne von Desmoglein 1 und/oder von Desmoglein 3 und/oder Varianten davon.

Ein weitere Aspekt der Erfindung richtet sich auf ein erfindungsgemäßes Fusionsprotein zur Verwendung in einem Verfahren zur Behandlung von Autoimmunerkrankungen. Das Verfahren umfasst bevorzugt den Schritt des In-Kontakt-Bringens von einer Patientenflüssigkeit (insbesondere Blut oder Gehirn-Rückenmarks-Flüssigkeit) mit dem Fusionsprotein. In weiteren bevorzugten Ausführungsformen wird die Autoimmunerkrankung Pemphigus vulgaris behandelt.

Im Folgenden wird die Erfindung anhand von Abbildungen und Ausführungsbeispielen ohne Beschränkung des allgemeinen Erfindungsgedankens näher erläutert:
**Abbildung 1****: synthetisch hergestellte, codonoptimierte Gensequenz von Xyn10A**
**Abbildung 2****: Primer zur Herstellung des Xyn10A-Genfragments**
**Abbildung 3****: Immunoblot-Analyse der Expression von Dsg3-Xyn10A**
   HEK293-Zellen wurden mit dem Plasmid transfiziert, das für **Dsg3-Xyn10A** codiert. Der Leervektor wurde für eine Kontrolltransfektion verwendet. 3 Tage nach Transfektion wurden die Zellen geerntet. Medium und lysierte Zellen wurden mit Hilfe einer SDS-PAGE aufgetrennt und anschließend geblottet. Der Nachweis des rekombinanten Fusionsproteins erfolgte mittels eines gegen Dsg3 gerichteten Kaninchenserums. Die Bande von Dsg3-Xyn10A ist bei etwa 100 kDa zu erkennen.

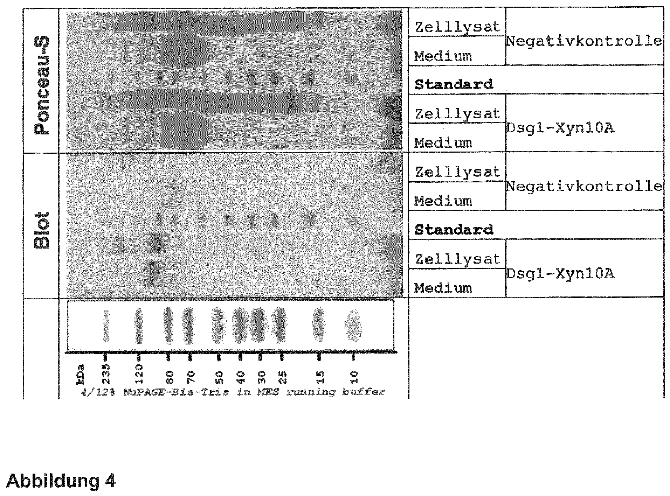

**Abbildung 4****: Immunoblot-Analyse der Expression von Dsg1-Xyn10A**
   HEK293-Zellen wurden mit dem Plasmid transfiziert, das für Dsg1-Xyn10A codiert. Der Leervektor wurde für eine Kontrolltransfektion verwendet. 3 Tage nach Transfektion wurden die Zellen geerntet. Medium und lysierte Zellen wurden mit Hilfe einer SDS-PAGE aufgetrennt und anschließend geblottet. Der Nachweis des rekombinanten Fusionsproteins erfolgte mittels eines gegen Dsg1 gerichteten Kaninchenserums. Die Bande von Dsg1-Xyn10A ist bei etwa 100 kDa zu erkennen.

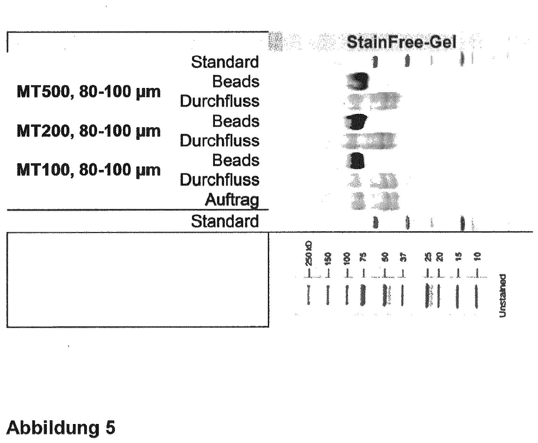

**Abbildung 5****: Gelanalyse der Bindung von Dsg3-Xyn10A an Zellulose-Beads**
   Verschiedene Zellulose-Beads wurden mit Dsg3-Xyn10A-haltigem Zellkulturüberstand mit Hilfe eines chromatografischen Verfahrens beladen. Je eine Probe des Auftrags, Durchflusses und der Beads wurde auf ein StainFree-Gel (Bio-Rad) aufgetragen und per SDS-PAGE getrennt. Nach Aktivierung durch UV-Licht wurden Tryptophan-haltige Proteine sichtbar. Die Bande von Dsg3-Xyn10 läuft bei etwa 100 kDa.

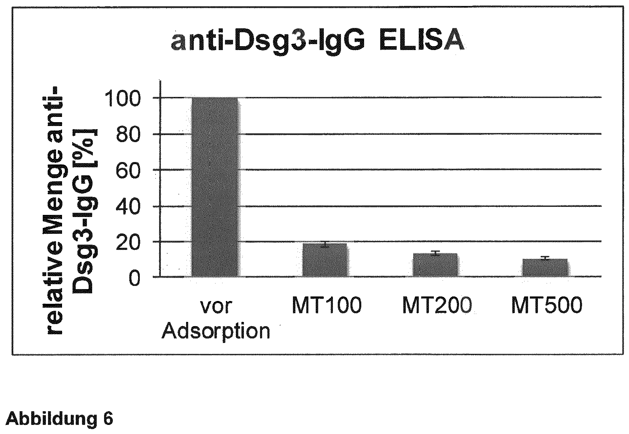

**Abbildung 6****: Gehalt an anti-Dsg3-IgG vor und nach Adsorption an mit Dsg3-Xyn10A-beladenen Zellulose-Beads**
   Ein Pool von Seren von Patienten mit Pemphigus vulgaris (PV) wurde mit Zellulose-Beads inkubiert, die vorher mit **Dsg3-Xyn10A** beladen worden waren. Das Volumenverhältnis von Serum zu Beads betrug 10:1. Nach 30-minütiger Inkubation wurde der Serenpool von den Beads getrennt, mit einem anti-Dsg3-IgG ELISA analysiert und mit dem Wert vor der Adsorption verglichen.
   Das Experiment wurde in Dreifachbestimmung durchgeführt. Die Fehlerindikatoren symbolisieren die Standardabweichung.

### Beispiele

### Beispiel 1: Verhalten verschiedener Zellulose-Beads bei unterschiedlichen Drücken und Flussraten in einer Chromatographie-Säule

Die Beladung der Zellulose-Beads mit dem rekombinant exprimierten Antigen-CBM-Fusionsprotein soll bevorzugt über ein chromatografisches Verfahren erfolgen. Hohe Flussraten ermöglichen dabei kürzere Prozesszeiten, gehen aber mit einem höheren Druck innerhalb der Säule einher. Um diesem Druck standzuhalten, müssen die Zellulose-Beads eine ausreichende mechanische Stabilität besitzen, die durch Quervernetzung der Polymerketten der Zellulose erreicht wird. Eine starke Quervernetzung eines Polymers führt jedoch immer zu einer Verkleinerung der vorhandenen Poren. Kleine Poren wiederum können das Eindringen großer Proteine in die Beads verhindern und so die Bindungskapazität des fertigen Immunadsorbers verringern.

**Tabelle 1: Zellulose-Beads und ihre Eigenschaften laut Herstellerangaben**

| **Name** | **Hersteller** | **Partikeldurchmesser** | **Porengröße** | **empfohlene Flussrate** | **maximaler Druck (Δp)** |
|---|---|---|---|---|---|
| **Cellufine GH-25** | **AMS** Biotechnology (Europe) Ltd - Deutschland | 40-130 µm | 3 kDa | 100-300 cm/h | 2 bar |
| **Cellufine GCL-2000** | AMS Biotechnology (Europe) Ltd - Deutschland | 40-130 µm | 3000 kDa | 5-50 cm/h | 1 bar |
| **lontosorb MT 100, 50-80 µm** | IONTOSORB | 50-80 µm | 100 kDa | max. 750 cm/h | 2,7 bar |
| **lontosorb MT 100, 80-100 µm** | IONTOSORB | 80-100 µm | 100 kDa | max. 750 cm/h | 2,7 bar |
| **lontosorb MT 200, 50-80 µm** | IONTOSORB | 50-80 µm | 200 kDa | max. 450 cm/h | 2 bar |
| **lontosorb MT 200, 80-100 µm** | IONTOSORB | 80-100 µm | 200 kDa | max. 450 cm/h | 2 bar |

Von den in Tabelle 1 aufgelisteten Zellulose-Beads wurden je 50 ml in eine HiScale 26/20-Säule (GE Healthcare) gepackt und mit Hilfe einer Äkta Pure Chromatografie-Anlage (GE Healthcare) mit Wasser gespült. Dabei wurden verschiedene Flussraten angelegt und beobachtet, wie sich der Druck entwickelt und ob sich das Gelbett (irreversibel) deformiert. Dabei wurde die Startflussrate für fünf Säulenvolumina beibehalten, um die Lagerungslösung vollständig aus der Beadsuspension zu waschen. Mit jedem weiteren Säulenvolumen wurde dann die Flussrate und damit der Druck erhöht.

**Tabelle 2: Chromatografie-Bedingungen für die Untersuchung der mechanischen Stabilität der Zellulose-Beads**

| **Beads** | **Startflussrate** | **Steigerung je Säulenvolumen** | **maximale Flussrate** |
|---|---|---|---|
| Cellufine GH-25 | 5 ml/min (57 cm/h) | 2 ml/min | 25 ml/min (283 cm/h) |
| Cellufine GCL-2000 | 3 ml/min (34 cm/h) | 0,5 ml/min | 4,5 ml/min (51 cm/h) |
| lontosorb MT 100, 50-80 µm | 5 ml/min (57 cm/h) | 2 ml/min | 25 ml/min (283 cm/h) |
| lontosorb MT 100, 80-100 µm | 5 ml/min (57 cm/h) | 2 ml/min | 25 ml/min (283 cm/h) |
| lontosorb MT 200, 50-80 µm | 5 ml/min (57 cm/h) | 2 ml/min | 25 ml/min (283 cm/h) |
| lontosorb MT 200, 80-100 µm | 5 ml/min (57 cm/h) | 2 ml/min | 25 ml/min (283 cm/h) |

Die getesteten Beads erfüllten die geforderten Spezifikationen.

### Beispiel 2: Klonierung der Fusionsproteinkonstrukte und Expression der Fusionsproteine

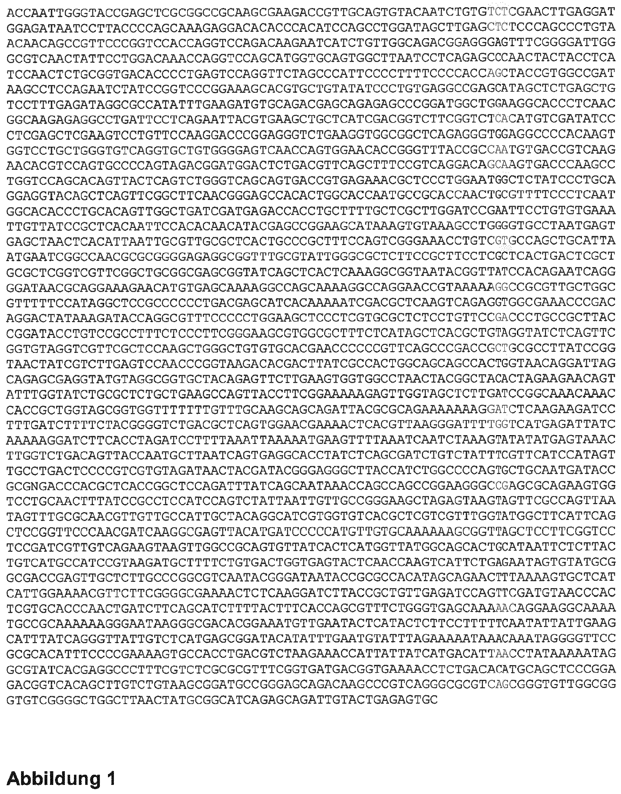

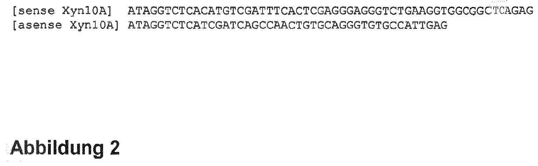

Die DNA-Sequenz für Xyn10A wurde in einer für menschliche Zellen Codon-optimierten Variante über Gensynthese hergestellt. Die Sequenz ist in Abbildung 1 zu sehen. Mittels PCR unter Verwendung der in Abbildung 2 angegebenen Primer wurde das Xyn10A-Genfragment vervielfältigt.

Das Xyn10A-Genfragments wurde gemeinsam mit einer Linker-Sequenz in ein Plasmid eingefügt, welches sowohl zur transienten als auch zur stabilen Expression in eukaryontischen Zellen verwendet werden kann. Die Linkersequenz soll verhindern, dass im fertigen Fusionsprotein das Autoantigen direkt in das Zellulose-bindende Modul übergeht und sich die beiden Funktionalitäten gegenseitig stören.

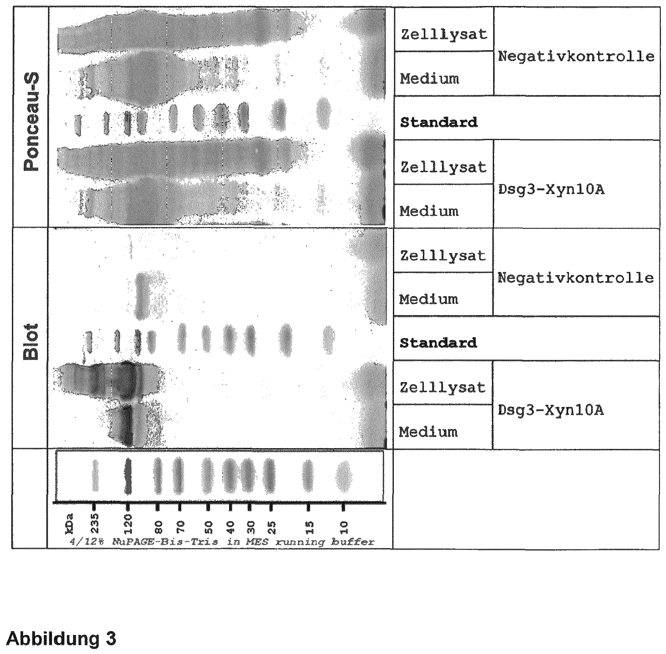

In einem zweiten Klonierungsschritt wurde die Gensequenz der Autoantigene vor der Linkersequenz in das Plasmid integriert. Die fertigen Expressionsplasmide wurden in geeignete Zellen transfiziert. Beispiele für die erfolgreiche Expression der rekombinanten Fusionsproteine sind in Abbildung 3 und Abbildung 4 dargestellt.

### Beispiel 3: Bindung eines Fusionsproteins an Zellulose-Beads

**Dsg3-Xyn10A** wurde stabil in HEK293-Zellen exprimiert. Der Zellkulturüberstand mit dem Zielprotein wurde mit verschiedenen Zellulose-Beads inkubiert. Anschließend wurden die Beads gewaschen und per SDS-PAGE analysiert (Abbildung 5).

Im Gel ist deutlich zu sehen, dass sich ein etwa 100 kDa großes Protein sehr stark auf den Beads angereichert hat. Die Identität dieses Proteins als **Dsg3-Xyn10A** wurde mit Hilfe eines Immunoblots bestätigt (nicht gezeigt). Andere Proteine haben nicht oder nur in sehr geringem Maße an die Zellulose-Beads gebunden.

### Beispiel 4: Adsorption von Autoantikörpern an mit Fusionsprotein beladenen Zellulose-Beads

Ein Pool aus Seren mehrerer Patienten mit Pemphigus vulgaris wurde an den mit Dsg3-Xyn10A-beladenen Zellulose-Beads adsorbiert. Mit Hilfe eines anti-Dsg3-IgG ELISAs wurde bestimmt, welcher Anteil der im Serenpool vorhandenen gegen Dsg3 gerichteten Antikörper entfernt werden konnte (Abbildung 6). Mehr als 80 % der vorhandenen Antikörper konnten durch die Adsorption an den Beads entfernt werden. Für diese Versuche wurden Zellulose-Beads mit einer Porengröße von 100 kDa (MT100), 200 kDa (MT200) und 500 kDa (MT500) verwendet. Es ist zu erkennen, dass Zellulose-Beads mit einer Porengröße von 200 kDa eine signifikant höhere Absorption zeigen als Zellulose-Beads mit einer Porengröße von 100 kDa.

## Patentansprüche

1. Immunadsorber zur Entfernung von Autoantikörpern mittels Autoantigen aus Flüssigkeiten, **dadurch gekennzeichnet, dass** der Immunadsorber Zellulose oder Derivate davon als Matrix aufweist und ein Ligand über eine Affinitätsbindung an die Matrix gebunden wird.

2. Immunadsorber gemäß Anspruch 1, wobei der Immunadsorber Zellulose-Beads als Matrix aufweist.

3. Immunadsorber gemäß einem der Ansprüche 1 oder 2, wobei die Affinitätsbindung über die Interaktion von Zellulose und Zellulose-bindenden Domänen des Liganden erfolgt.

4. Immunadsorber gemäß einem der Ansprüche 1 bis 3, wobei der Ligand als Fusionsprotein vorliegt und eine Zellulose-bindende Domäne und eine Autoantikörper-bindende Domäne aufweist.

5. Immunadsorber gemäß Anspruch 4, wobei die Autoantikörper-bindende Domäne ein Autoantigen zur Adsorption von Autoantikörpern, die mit einer Autoimmunerkrankung assoziiert sind, aufweist.

6. Immunadsorber gemäß einem der Ansprüche 3 bis 5, wobei das Autoantigen über die Zellulose-bindende Domäne an die Zellulose immobilisiert wird und die Autoantikörper-bindende Domäne des Autoantigens so präsentiert wird, dass Autoantikörper hieran binden können.

7. Immunadsorber gemäß einem der vorangegangenen Ansprüche, wobei das Autoantigen ausgewählt ist aus der Gruppe umfassend Desmoglein 1, Desmoglein 3, Bullöses-Pemphigoid-Antigen 2/Kollagen XVII, Aquaporin 4, NMDA-Rezeptor Subtyp 1 a, Contactin-assoziiertes Protein 2, Leucin-reiches, in Gliomen inaktiviertes Protein 1, Acetycholinrezeptor, Myelin-assoziiertes Glykoprotein und Varianten davon.

8. Immunadsorber gemäß einem der Ansprüche 3 bis 7, wobei die Zellulose-bindende Domäne ausgewählt ist aus der Gruppe bestehend aus Xyn10A aus Xylanase 10B aus *Cellulomonas fimi* gemäß SEQ ID NO:1.

9. Immunadsorber gemäß einem der vorangegangenen Ansprüche zur Verwendung in der Apherese.

10. Immunadsorber gemäß einem der vorangegangenen Ansprüche zur Verwendung bei der Behandlung und Prophylaxe von Autoimmunerkrankungen ausgewählt aus der Gruppe umfassend Pemphigus foliaceus, Pemphigus vulgaris, Bullöses Pemphigoid, Schleimhautpemphigoid, Neuromyelitis optica, longitudinale extensive transverse Myelitis, rezidivierende Optikusneuritis, NMDA-Rezeptor-Enzephalitis, limbische Enzephalitis, Neuromyotonie, Morvan-Syndrom, Myasthenia gravis, paraproteinnämische anti-MAG Gammopathie (auch monoklonale IgM-Gammopathie mit anti-MAG-Reaktivität, paraproteinämische Neuropathie) und/oder dilative Kardiomyopathie und zur Verwendung bei der Behandlung einer Antikörper-vermittelten Transplantatabstoßung.

11. Verfahren zur Herstellung eines Immunadsorbers gemäß einem der vorangegangenen Ansprüche zur Adsorption von Autoantikörpern mittels Autoantigen, umfassend die folgenden Schritte:
a) Bereitstellen eines Trägermaterials,
b) Immobilisieren des Liganden auf diesem Trägermaterial, wobei der Ligand über eine Affinitätsbindung an das Trägermaterial gebunden wird.

12. Verfahren zur Herstellung eines Immunadsorbers gemäß Anspruch 11, **dadurch gekennzeichnet, dass**
a) das Trägermaterial Zellulose aufweist und
b) die Immobilisation des Liganden über die Interaktion von Zellulose und Zellulose-bindenden Domänen erfolgt.

13. Verfahren gemäß Anspruch 12, wobei als Trägermaterial Zellulose-Beads verwendet werden.

14. Verfahren gemäß einem der Ansprüche 11 bis 13, wobei der Ligand als Fusionsprotein vorliegt, umfassend eine Zellulose-bindende Domäne und ein Autoantigen zur Adsorption von Autoantikörpern.

15. Verfahren gemäß einem der Ansprüche 11 bis 14, wobei das Trägermaterial und/oder der Immunadsorber vor und/oder nach der Beladung mit dem Liganden sterilisiert werden.

16. Ein Fusionsprotein umfassend eine Zellulose-bindende Domäne und eine Autoantikörper-bindende Domäne zur Verwendung in einem Verfahren zur Behandlung von Autoimmunerkrankungen.


SEQUENCE LISTING

<110> EUROIMMUN Medizinische Labordiagnostika AG

<120> Zellulose-basierter Immunadsorber

<130> 16PP139EP

<160> 3

<170> BiSSAP 1.3

<210> 1
<211> 3428
<212> DNA
<213> Artificial Sequence

<220>
<223> synthetisch hergestellte, codonoptimierte Gensequenz von Xyn10A

<400> 1
accaattggg taccgagctc gcggccgcaa gcgaagaccg ttgcagtgta caatctgtgt 60

ctcgaacttg aggatggaga taatccttac cccagcaaag aggacacacc cacatccagc 120

ctggatagct tgagctctcc cagccctgta acaacagccg ttcccggtcc accaggtcca 180

gacaagaatc atctgttggc agacggaggg agtttcgggg attgggcgtc aactattcct 240

ggacaaacca ggtccagcat ggtgcagtgg cttaatcctc agagcccaac tactacctca 300

tccaactctg cggtgacacc cctgagtcca ggttctagcc cattcccctt ttccccacca 360

gctaccgtgg ccgataagcc tccagaatct atccggtccc ggaaagcacg tgctgtatat 420

ccctgtgagg ccgagcatag ctctgagctg tcctttgaga taggcgccat atttgaagat 480

gtgcagacga gcagagagcc cggatggctg gaaggcaccc tcaacggcaa gagaggcctg 540

attcctcaga attacgtgaa gctgctcatc gacggtcttc ggtctcacat gtcgatatcc 600

ctcgagctcg aagtcctgtt ccaaggaccc ggagggtctg aaggtggcgg ctcagagggt 660

ggaggcccca caagtggtcc tgctgggtgt caggtgctgt ggggagtcaa ccagtggaac 720

accgggttta ccgccaatgt gaccgtcaag aacacgtcca gtgccccagt agacggatgg 780

actctgacgt tcagctttcc gtcaggacag caagtgaccc aagcctggtc cagcacagtt 840

actcagtctg ggtcagcagt gaccgtgaga aacgctccct ggaatggctc tatccctgca 900

ggaggtacag ctcagttcgg cttcaacggg agccacactg gcaccaatgc cgcaccaact 960

gcgttttccc tcaatggcac accctgcaca gttggctgat cgatgagacc acctgctttt 1020

gctcgcttgg atccgaattc ctgtgtgaaa ttgttatccg ctcacaattc cacacaacat 1080

acgagccgga agcataaagt gtaaagcctg gggtgcctaa tgagtgagct aactcacatt 1140

aattgcgttg cgctcactgc ccgctttcca gtcgggaaac ctgtcgtgcc agctgcatta 1200

atgaatcggc caacgcgcgg ggagaggcgg tttgcgtatt gggcgctctt ccgcttcctc 1260

gctcactgac tcgctgcgct cggtcgttcg gctgcggcga gcggtatcag ctcactcaaa 1320

ggcggtaata cggttatcca cagaatcagg ggataacgca ggaaagaaca tgtgagcaaa 1380

aggccagcaa aaggccagga accgtaaaaa ggccgcgttg ctggcgtttt tccataggct 1440

ccgcccccct gacgagcatc acaaaaatcg acgctcaagt cagaggtggc gaaacccgac 1500

aggactataa agataccagg cgtttccccc tggaagctcc ctcgtgcgct ctcctgttcc 1560

gaccctgccg cttaccggat acctgtccgc ctttctccct tcgggaagcg tggcgctttc 1620

tcatagctca cgctgtaggt atctcagttc ggtgtaggtc gttcgctcca agctgggctg 1680

tgtgcacgaa ccccccgttc agcccgaccg ctgcgcctta tccggtaact atcgtcttga 1740

gtccaacccg gtaagacacg acttatcgcc actggcagca gccactggta acaggattag 1800

cagagcgagg tatgtaggcg gtgctacaga gttcttgaag tggtggccta actacggcta 1860

cactagaaga acagtatttg gtatctgcgc tctgctgaag ccagttacct tcggaaaaag 1920

agttggtagc tcttgatccg gcaaacaaac caccgctggt agcggtggtt tttttgtttg 1980

caagcagcag attacgcgca gaaaaaaagg atctcaagaa gatcctttga tcttttctac 2040

ggggtctgac gctcagtgga acgaaaactc acgttaaggg attttggtca tgagattatc 2100

aaaaaggatc ttcacctaga tccttttaaa ttaaaaatga agttttaaat caatctaaag 2160

tatatatgag taaacttggt ctgacagtta ccaatgctta atcagtgagg cacctatctc 2220

agcgatctgt ctatttcgtt catccatagt tgcctgactc cccgtcgtgt agataactac 2280

gatacgggag ggcttaccat ctggccccag tgctgcaatg ataccgcgng acccacgctc 2340

accggctcca gatttatcag caataaacca gccagccgga agggccgagc gcagaagtgg 2400

tcctgcaact ttatccgcct ccatccagtc tattaattgt tgccgggaag ctagagtaag 2460

tagttcgcca gttaatagtt tgcgcaacgt tgttgccatt gctacaggca tcgtggtgtc 2520

acgctcgtcg tttggtatgg cttcattcag ctccggttcc caacgatcaa ggcgagttac 2580

atgatccccc atgttgtgca aaaaagcggt tagctccttc ggtcctccga tcgttgtcag 2640

aagtaagttg gccgcagtgt tatcactcat ggttatggca gcactgcata attctcttac 2700

tgtcatgcca tccgtaagat gcttttctgt gactggtgag tactcaacca agtcattctg 2760

agaatagtgt atgcggcgac cgagttgctc ttgcccggcg tcaatacggg ataataccgc 2820

gccacatagc agaactttaa aagtgctcat cattggaaaa cgttcttcgg ggcgaaaact 2880

ctcaaggatc ttaccgctgt tgagatccag ttcgatgtaa cccactcgtg cacccaactg 2940

atcttcagca tcttttactt tcaccagcgt ttctgggtga gcaaaaacag gaaggcaaaa 3000

tgccgcaaaa aagggaataa gggcgacacg gaaatgttga atactcatac tcttcctttt 3060

tcaatattat tgaagcattt atcagggtta ttgtctcatg agcggataca tatttgaatg 3120

tatttagaaa aataaacaaa taggggttcc gcgcacattt ccccgaaaag tgccacctga 3180

cgtctaagaa accattatta tcatgacatt aacctataaa aataggcgta tcacgaggcc 3240

ctttcgtctc gcgcgtttcg gtgatgacgg tgaaaacctc tgacacatgc agctcccgga 3300

gacggtcaca gcttgtctgt aagcggatgc cgggagcaga caagcccgtc agggcgcgtc 3360

agcgggtgtt ggcgggtgtc ggggctggct taactatgcg gcatcagagc agattgtact 3420

gagagtgc 3428

<210> 2
<211> 56
<212> DNA
<213> Artificial Sequence

<220>
<223> Primer zur Herstellung des Xyn10A-Genfragments, sense Xyn10A

<400> 2
ataggtctca catgtcgatt tcactcgagg gagggtctga aggtggcggc tcagag 56

<210> 3
<211> 44
<212> DNA
<213> Artificial Sequence

<220>
<223> Primer zur Herstellung des Xyn10A-Genfragments, asense Xyn10A

<400> 3
ataggtctca tcgatcagcc aactgtgcag ggtgtgccat tgag 44